# EUROPEAN PATENT SPECIFICATION

(11) **EP 3 331 369 B1**
(45) Date of publication and mention of the grant of the patent: **10.11.2021**
(21) Application number: 16835563.4
(22) Date of filing: 25.05.2016
(51) Int. Cl.: A23C 3/07, A23C 3/00, A61L 2/00, A61L 2/08, A61L 2/10

(54) **METHODS FOR PRODUCTION OF AN IMMUNE-ACTIVE MILK PRODUCT AND USES THEREOF**
VERFAHREN ZUR HERSTELLUNG VON IMMUNAKTIVEM MILCHPRODUKT UND VERWENDUNGEN DAVON
PROCÉDÉS DE PRODUCTION D'UN PRODUIT LAITIER IMMUNOACTIF ET UTILISATIONS ASSOCIÉES

(30) Priority: 07.08.2015 US 201562202173 P
(43) Date of publication of application: 13.06.2018
(73) Proprietor: Tamarack Biotics LLC, Fresno, California 93711 (US)
(72) Inventor: COMSTOCK, Robert L., Fresno, California 93711 (US)
(74) Representative: CSY London
(86) International application number: PCT/US2016/034128
(87) International publication number: WO 2017/027081

(56) References cited:
- WO-A1-2013/011040
- WO-A1-2014/094189
- US-A1- 2004 208 955
- US-B1- 6 391 362
- CHATRAPATI V.R.K. TAMMINEEDI ET AL: "Determining the effect of UV-C, high intensity ultrasound and nonthermal atmospheric plasma treatments on reducing the allergenicity of [alpha]-casein and whey proteins", LWT- FOOD SCIENCE AND TECHNOLOGY, vol. 54, no. 1, 1 November 2013 (2013-11-01), pages 35-41, XP055546254, United Kingdom ISSN: 0023-6438, DOI: 10.1016/j.lwt.2013.05.020
- GEORG LOSS ET AL: "The protective effect of farm milk consumption on childhood asthma and atopy: The GABRIELA study", JOURNAL OF ALLERGY AND CLINICAL IMMUNOLOGY, vol. 128, no. 4, 1 October 2011 (2011-10-01), pages 766-773.e4, XP055546255, AMSTERDAM, NL ISSN: 0091-6749, DOI: 10.1016/j.jaci.2011.07.048
- LUKAS CHRISTEN ET AL: "The Effect of UV-C Pasteurization on Bacteriostatic Properties and Immunological Proteins of Donor Human Milk", PLOS ONE, vol. 8, no. 12, 23 December 2013 (2013-12-23), page e85867, XP055546257, DOI: 10.1371/journal.pone.0085867
- SARA SCHAEFER ET AL: "Use of UV Treated Milk Powder to Increase Vaccine Efficacy in the Elderly", FRONTIERS IN IMMUNOLOGY, vol. 9, 17 October 2018 (2018-10-17), XP055546256, DOI: 10.3389/fimmu.2018.02254
- FREEMAN ET AL.: 'Dairy proteins and the response to pneumovax in senior citizens: a randomized, double-blind, placebo-controlled pilot study' ANNALS OF THE NEW YORK ACADEMY OF SCIENCES, [Online] vol. 1190, 2010, pages 97 - 103, XP055363500 Retrieved from the Internet: <URL:https://www.researchgate.net/profiie/P atrick_Leung/pubtication/43133221_Dairy_pro teins_and_t he_response_to_pneumovax_in_senior_eitizens _A_randomized_double-blind_placebo-controll e d_pilot_study/links/00463513f7f4f7649900000 0.pdf> [retrieved on 2016-07-20]
- DUGCAL: 'Whey Proton's Impact on Mood and Stress' INSIDETRACKER, [Online] 13 November 2014, XP055363505 Retrieved from the Internet: <URL:http://blog.insidetracker.com/whey-pro teins-impact-on-mood-and-stress> [retrieved on 2016-07-20]

## Description

### TECHNICAL FIELD

The present invention relates to a processing method to produce an immune active milk product and methods of using the immune active milk product to enhance immune response in individuals.

### BACKGROUND

Asthma and allergic diseases continue to expand in the developed world and are growing rapidly in the developing world. Prevention has remained elusive despite years of research.

Immunosenescence describes the gradual degradation of immune activity brought on through aging. An impaired function of both the innate and acquired arms of the immune system is observed, and results in a higher susceptibility to infectious disease. The use of vaccines is recommended in senior citizens, however, a successful immune response to the vaccine is observed only in an age-dependent proportion of cases. Poor response to vaccines is a common manifestation of immunosenescence. A recent study demonstrated that whey proteins improved vaccine response in an elderly population.

Stress induced immune suppression, or "Olympic Village Fever" as it is commonly known, has demonstrated a link between high levels of exercise induced stress and greater susceptibility to communicable diseases. Additionally, it is desirable to promote muscle recovery after exercise, particularly intense exercise.

A number of studies have showed a correlation between decreased allergy and raw milk consumption. Loss et al. in "The protective effect of farm milk consumption on childhood asthma and atopy: The GABRIELA study" in Allergy and Clinical Immunology Vol. 128, Num. 4 (2014) reported that questionnaire-reported consumption of unboiled farm milk was inversely associated with asthma, hay fever, and atopy. Higher levels of the whey proteins BSA, a- lactalbumin, and β-lactoglobulin in milk samples were associated with a reduced risk of asthma. Neither total viable bacterial counts nor the total fat content of the milk were related to asthma or atopy.

Many clinical studies have demonstrated a link between raw milk consumption and reduced allergy development, in particular, the PASTURE study, Loss, et al. "Consumption of unprocessed cow's milk protects infants from common respiratory infections," Journal of Allergy and Clinical Immunology, 2015 Jan;135(1):56-62,demonstrated raw milk consumption and not farm life exposure were responsible for the observed allergy and respiratory infection reduction. The study's conclusions stated that early life consumption of raw cow's milk reduced the risk of manifest respiratory infections and fever by about 30%.

Further a study in 2013 (Christen et al., "The Effect of UV-C Pasteurization on Bacteriostatic and Immunological Proteins of Donor Human Milk", PLOS ONE, 8(12) e85867, showed that human milk's levels of immunological proteins is significantly higher under UV-C irradiation versus Holder Pasteurization.

However, raw cow's milk is capable of transmitting serious infections, such as tuberculosis, brucellosis, listeriosis, or enterohemorrhagic E. coli. Introduction of pasteurization and other processing techniques minimized the chances of milk-borne infections. The PASTURE study noted that if the health hazards of raw milk could be overcome, the public health impact of minimally processed but pathogen- free milk might be enormous, given the high prevalence of respiratory infections in the first year of life and the associated direct and indirect costs.

Bovine milk is a complicated biofluid containing a multitude of carbohydrates, proteins and lipids. Various compounds have been proposed as being responsible for raw milk's protective effects, but no study so far conducted has demonstrated effects as strong as those observed through raw milk consumption. Milk fat globule membrane (MFGM) describes a range of proteins and phospholipids that surround milk's fat globules. These compounds contain a great variety of proteins, many of which have demonstrated immune activity. MFGM components (which includes some or all of the milkfat globule and attendant proteins, phospholipids, and triglycerides) has been successfully extracted and isolated from milk and milk derivatives such as buttermilk.

Research has demonstrated that many of bovine raw milk' s compounds stimulate human immune systems, however, thermal pasteurization degrades many of these immune active compounds. New methods are required to enable many of these immune active compounds to survive a pasteurization process that continues to meet the required 5 log reduction in pathogens.

Lactose intolerance affects a large percentage of the world's population. Additionally, many elderly people, despite no previous intolerance, develop this condition as they age. Enzymatically hydrolyzing lactose into glucose and galactose is a well-known process and reduces lactose levels by at least 95%. Other methods for removing lactose, glucose and/or galactose are known in the art, including ultrafiltration.

The present invention is directed toward overcoming one or more of the problems discussed above.

### PRIOR ART

Food Science and Technology 54(1), 2013, 35-41 relates to an investigation of the effect of high intensity ultrasound, nonthermal atmospheric plasma and UV-C light treatments in reducing the allergenicity of isolated major milk proteins.

Journal of Allergy and Clinical Immunology 128(4), 2011, 766 ― 773 suggests that the protective effect of raw milk consumption on asthma might be associated with the whey protein fraction of milk.

PLOS ONE 8(12), 2013, page e85867 discloses a study investigating the bacterial growth rate and the immunological protein concentration of ultraviolet (UV-C) irradiated, Holder pasteurized and untreated human milk.

Front. Immunol., 17 October 2018 relates to a controlled, randomized, double blind pilot study was conducted to compare two different protein sources (soy and dairy) as nutritional supplementation to enhance vaccine response.

WO2014094189A1 relates to a method and apparatus for the inactivation of a biological contaminant in a human milk product by exposing the milk product to UV by imparting a vortical flow to the milk product. The exposure is stated to inactivate or reduce the amount of a contaminant in the milk product, whilst the activity of a bioactive component present in the milk product is not substantially reduced.

US6391362B1 relates to a bulk preparation method for isolating milk fat globule membranes from milk in which the separation method is the same whether the milk fat globule membranes are collected in bulk in the dairy industry or collected for an individual patient for diagnostic purposes.

US2004208955A1 describes a dried enhanced-solubility milk protein concentrate (MPC) containing at least one monovalent salt added prior to drying. Methods for preparing this product are provided.

WO2013011040A1 describes a raw milk preparation for use for treating or preventing childhood asthma and allergic diseases and methods of producing the raw milk preparation for use for treating or preventing childhood asthma and allergic diseases.

Annals of the New York Academy of Sciences, 1190 (2010) 97 ― 103 describes a controlled, randomized, double-blind pilot study to determine if an eight-week supplementation with whey protein (or soy protein used as control) could enhance the serum response to pneumococcal vaccine in healthy senior citizens.

### SUMMARY OF THE EMBODIMENTS

The present invention includes a method to produce an immune-active milk product. The method includes steps of
a. obtaining a nonfat milk fraction from raw (unpasteurized) milk;
b. obtaining a composition comprising milk fat globule membrane components (MFGM) from buttermilk;
c. combining the composition comprising MFGM components with the nonfat milk fraction; and
d. pasteurizing the product of step c) by application of UV-C light to prepare the immune-active milk product.

According to a second embodiment, the invention relates to an immune-active milk product produced by the method of the invention.

According to a third embodiment, the invention relates to immune-active milk product obtained by the process of the invention for use in the treatment of childhood asthma.

MFGM is obtained from buttermilk, by methods known in the art, and added to the nonfat milk fraction in liquid form.

In one embodiment, the nonfat milk fraction is treated to lower the levels of sugar, e.g., lactose, galactose, and/or glucose content. In one embodiment, the sugar is removed via hydrolysis of the lactose before or after the UV-C pasteurization step. In another embodiment, the sugar is removed via a mechanical process such as, for example, ultrafiltration.

In another embodiment, the immune-active milk product is dried to a powder using a low-heat drying process. In one embodiment, the drying step is a freeze-drying step. In another embodiment, the drying step occurs below 45 °C. Alternatively, a combination of ultrafiltration and diafiltration may be used to create a milk protein concentrate.

In one embodiment, the UV-C light is applied at between 1000-2000 J/liter, or as much as necessary to achieve a 5-log reduction of bacterial content in the composition.

There is herein described a method for reducing childhood asthma and allergic diseases, which includes administering an immune-active milk product obtained by the processes of the instant invention to a patient in need thereof.

There is herein described a method for reducing elderly immunosenescence, which includes administering an immune-active milk product obtained by the processes of the instant invention to a patient in need thereof.

There is herein described a method for reducing stress-induced immune suppression, which includes administering an immune-active milk product obtained by the process processes of the instant invention to a patient in need thereof. Stress-induced immune suppression may be brought on by, for example, exercise. The compositions of the present invention may also be used to enhance muscle recovery after, for example, exercise.

The present invention also includes a method to produce an immune-active milk product which includes the steps of obtaining a nonfat milk fraction from raw (unpasteurized) milk; adding a composition comprising MFGM components from buttermilk, pasteurizing the product by application of UV-C light, drying the pasteurized nonfat milk fraction or alternatively, creating a milk protein concentrate by ultrafiltration/diafiltration to prepare the immune-active milk product.

### BRIEF DESCRIPTION OF THE DRAWINGS

A further understanding of the nature and advantages of particular embodiments may be realized by reference to the remaining portions of the specification and the drawings, in which like reference numerals are used to refer to similar components. In some instances, a sub- label is associated with a reference numeral to denote one of multiple similar components. When reference is made to a reference numeral without specification to an existing sub-label, it is intended to refer to all such multiple similar components.
Fig. 1 shows LC/MS TIC chromatograms of the raw milk samples (first and second chromatograms) and 1000 J-treated samples (third to sixth chromatograms.)
Fig. 2 shows relative levels of a number of protease inhibitors in the raw milk samples, conventionally spray dried samples, pasteurized samples and 1000 J samples (1x or 2x).
Fig. 3 shows relative levels of several complement proteins in the raw milk samples, conventionally spray dried samples, pasteurized samples and 1000 J samples (1x or 2x).
Fig. 4 shows relative levels of other immune-active proteins in the raw milk samples, conventionally spray dried samples, pasteurized samples and 1000 J samples (1x or 2x).
Figure 5 shows a flow chart of the process to prepare an immune-active milk product of the present invention.
Figure 6 shows a flow chart of the process to prepare an immune-active milk product of the present invention.
Figure 7 shows a flow chart of the process to prepare an immune-active milk product of the present invention.

### DETAILED DESCRIPTION

The present invention allows for the production and use of a product that shares bovine raw milk's ability to stimulate human immune systems, without the infectious disease risk of bovine raw milk. The present invention has developed new methods to treat raw milk to allow for immunologically active proteins and other components such as enzymes, antibodies, and the like to undergo a non-thermal pasteurization process that continues to meet the required 5-log reduction in pathogens of marketed milk. The methods of the invention avoid the use of thermal pasteurization and conventional spray drying, which have been found to reduce the amount and/or quality of immunologically active proteins in milk. For example, the methods can include a low temperature step which exposes milk to a maximum temperature of 42°C for less than 2 minutes, which preserves immunologically active proteins.

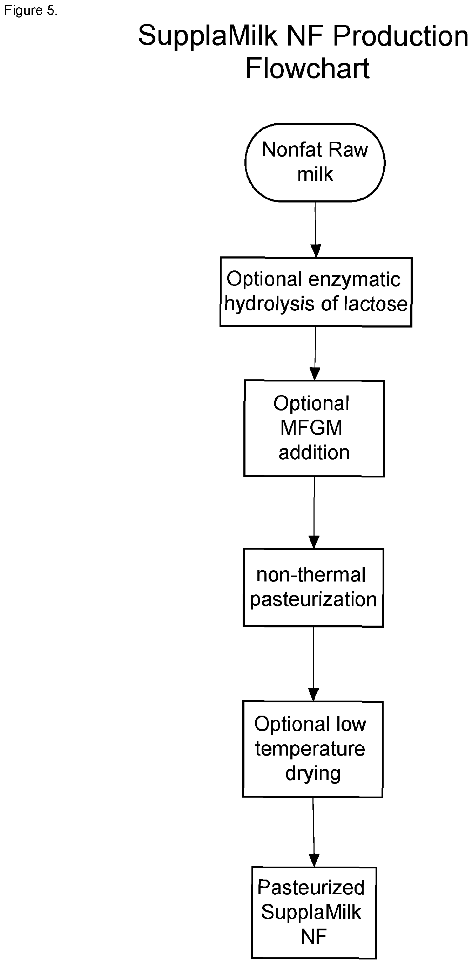

Therefore, in one embodiment, the present invention includes a method to produce immune-active milk products. The invention includes obtaining a raw milk composition, separating the raw milk composition into a nonfat milk fraction and a fat fraction, and pasteurizing the nonfat milk fraction by application of UV-C light (non-thermal pasteurization) to prepare the immune-active milk product. The MFGM component is added to the nonfat milk fraction prior to non-thermal pasteurization. Figure 5 shows an embodiment of the process described by the instant invention.

Step 1 of Figure 5 involves obtaining a nonfat raw milk fraction from a raw milk composition. Raw milk is generally obtained whole from the dairy animal, and raw whole milk suitable for the invention includes milk which has not been thermally processed prior to use according to the methods disclosed herein. Generally, raw milk is milk that has not been pasteurized or homogenized. Raw milk can be obtained from, for example, dairy animals, such as cattle, buffaloes, goats, sheep and camels, or other milk animals such as yaks, horses, reindeer and donkeys. In one embodiment, the dairy animal is a cow. Alternatively, a nonfat milk fraction from raw milk may also be obtained.

In one embodiment, if the starting material is raw whole milk, a nonfat milk fraction is separated from the fat fraction of the milk. Such separation is routine in the art and can be accomplished by any method known in the art. For example, centrifugation is a standard operation used in the food industry for the treatment of milk. Centrifugation uses centrifugal force to separate two mixed liquids or insoluble solids from liquids (suspension). For milk, the fat globules separate from the rest of the milk suspension. The original milk feed at about 3.7% fat is separated into a cream portion (higher than 30% fat) and a skim milk portion (around 0.05% fat). The milk is commonly at about 40°C before entering the centrifuge for optimized liquidity of the fat. Commonly a lower-speed centrifugation step at about 5,000-10,000 x g is used.

Optionally, the nonfat milk fraction may be treated to lower the amount of sugar present in the milk fraction. Sugars include lactose, glucose and/or galactose. In one embodiment, removal of sugar is accomplished via hydrolysis of lactose, according to Step 2 of Figure 5, although the step comprising hydrolysis of lactose may occur at any point along the process. Optionally, this step occurs after the non-thermal pasteurization e.g. UV-C pasteurization step described below, or may be performed prior to the non-thermal pasteurization step. If lactose hydrolysis is performed after the non-thermal pasteurization step, a sterile lactase preparation should be used. Lactose intolerance is the inability of many people to digest lactose, which leads to symptoms such as abdominal bloating, cramps, flatulence, and diarrhea in individuals lacking sufficient amounts of the enzyme lactase. It is estimated that approximately 70% of the world's population is intolerant to some extent to lactose. Cow's milk has about 5% lactose. Lactase, or β-galactosidase, can be used to break lactose down into galactose and glucose monomers. Direct treatment of milk using lactase is known in the art and can be accomplished by any method as known in the art. For example, lactase from a source such as fungi of the genus Aspergillus or yeast of the genus Kluyveromyces may be mixed in with the product to break down the lactose in the milk. An example of a suitable lactase is LACTOZYM PURE (Novozymes), and amounts to use, temperatures and procedures to accomplish the suitable amount of hydrolysis of lactose are in accordance with the manufacturer's directions.

An additional component, e.g., a MFGM-containing composition, is added to the nonfat milk fraction before the UV-C pasteurization step to prepare the immune-active milk product of the invention, according to Step 3 of Figure 5. The MFGM-containing composition is obtained from buttermilk. Milk fat is primarily triglycerides and phospholipids and is secreted from mammary epithelial cells as fat globules, primarily composed of a globule of triglyceride surrounded by a lipid bilayer membrane which helps to stabilize the fat globules in the milk as an emulsion. The milk fat globule includes the milk fat and the associated milk fat globule membrane. The milk fat globule membrane (MFGM) describes a range of proteins and phospholipids that surround milk fat globules. The term MFGM components defines a composition which is enriched in some or all of the protein components of the milk fat globule membrane. These milk fat globule proteins contain a great variety of proteins, many of which have demonstrated immune activity. The milk fat globule membrane protects the milk fat from naturally occurring lipase enzymes and the development of rancid off-flavors. In conventional heat pasteurization steps, lipase enzymes are inactivated by heat during heat pasteurization which slows down the rancidity process. However, in the processes of the instant invention, enzymes are not inactivated to the same degree.

The MFGM-containing composition suitable for use with the present invention may comprise MFGM components. MFGM components may be obtained from buttermilk by methods as known in the art. For example, whole milk is generally 10% cream (4-5% fat). The cream may be churned into butter with a remaining buttermilk that contains less lipids and phospholipids and enhanced MFGM components as compared to the starting cream or whole milk. The buttermilk contains water and is enriched for MFGM as compared to the starting cream. The buttermilkis used as the source of MFGM and may be added to the nonfat milk portion such that the ratio of MFGM is approximately the same as in raw milk. For example, the buttermilk can be added back to the nonfat milk at a 5% v/v ratio. Generally, preparation processes for MFGM separate most or all of the fat (triglycerides and phospholipids, for example) from the remainder of the components, such that when blended with the nonfat milk fraction or the non-thermally pasteurized e.g., UV-C pasteurized nonfat milk fraction of the present invention, approximates the milkfat level as desired, such as, in some cases to be equivalent to approximately 1-2% fat.

When MFGM components are not obtained commercially, they may be treated by the non-thermal pasteurization methods as described herein, either prior to or after combination with the nonfat milk portion prepared according to the present invention.

Compositions comprising MFGM components may also be obtained commercially, from, for example, Aria Foods Ingredients Group P/S (Denmark), LACPRODAN MFGM- 10 (whey protein concentrate). This material is described as a whey protein concentrate with a high concentration of bioactive proteins and lipid. Typical batches of LACPRODAN MFGM-10 have a total fat composition of 14-20%, phospholipids of 6-8%, with an IgG level of 4%, lactoferrin of 0.08%, lactose maximum 3%, and sphingomyelin of 1.6%, phosphatidyl ethanolamine of 1.9%, phosphatidyl choline of 1.8%, phosphatidyl inositol of 0.5%, and phosphatidyl serine level of about 0.8%. Other sources of a protein/phospholipid-containing composition include Fonterra, Inc. (New Zealand). MFGM components are also available from commercial suppliers, or may be prepared by methods known in the art, see, e.g., MacGibbon AKH and Taylor MW (2006) Composition and Structure of Bovine Milk Lipids in Advanced Dairy Chemistry Volume 2, Lipids, 3rd Ed, (P.F. Fox and P.L.H. McSweeney, eds.) Springer, New York, pp 1-43. Protein/phospholipid-containing compositions comprising MFGM components may be pasteurized by conventional methods, such as, for example, heat-pasteurization, or optionally, by UV-C pasteurization. For example, MFGM components obtained from commercial sources may have been heat-pasteurized.

The protein/phospholipid-containing composition comprising MFGM components can be added back to the nonfat fraction of the raw milk compositionThe MFGM containing composition is added back to the nonfat milk fraction prior to the UV-C pasteurization step of the nonfat milk fraction.

The present invention also includes a non-thermal pasteurization of the nonfat milk fraction, according to Step 4 of Figure 5. The non-thermal pasteurization includes a UV-C pasteurization. Methods to accomplish a UV-C treatment to accomplish a suitable pasteurization are known in the art. According to the U.S. Food and Drug Administration, milk requires a 5-log₁₀ bacterial reduction. Methods to obtain such a reduction in bacterial using UV-C are known in the art. Generally, ultraviolet (UV) irradiation is classified as a non-thermal disinfection method. UV is subdivided by wavelength into UV-A (320-400 nm), UV-B (280-320 nm), UV-C (200-280 nm) and Vacuum-UV (100-200 nm). UV-C has the highest germicidal effect, specifically between 250 and 270 nm, and is capable of destroying bacteria, viruses, protozoa, yeasts, molds and algae. The penetration depth of UV-C depends on the solubility, density and turbidity of a liquid. Milk is difficult to treat with UV-C due to its high absorption coefficient of 300 cm⁻¹ at a wavelength of 254 nm. Previous studies showed that UV- C irradiation can be used to successfully reduce the microbial load of opaque liquids such as bovine milk, for example, by creating a turbulent flow around a UV-C source and this therefore exposed the micro-organisms to photons at the interface between the opaque liquid and the photon source. Key factors influencing efficiency of UV-C treatment include reactor design, fluid dynamic parameters and UV-C absorbance of liquid food (Koutchama et al., 2004). Turbulent flow of liquid foods in continuous flow UV reactors increased inactivation of microorganisms in fresh juices, liquid egg whites and milk (Koutchma et al., 2004; Franz et al., 2009).

An example of a UV suitable for the present invention includes that of Choudhary et al. (2011), Performance of coiled tube ultraviolet reactors to inactivate Escherichia coli W1485 and Bacillus cereus endospores in raw cow milk and commercially processed skimmed cow milk. Journal of Food Engineering, 107 (1): 14-20. Choudhary et al. (2011) reported a 4.1 log₁₀ CFU/ml reduction of E. coli W 1485 in raw cow milk by UV-C treatment in a coiled tube UV reactor at a flow rate of 100 1pm, corresponding to a Rₑ(Reynolds number)of 713 at a UV dose of 11.187 mJ/cm².

Another example of UV-C device suitable for the present invention can be found in U.S. 6,916,45. A dose rate of 2000 J/1 has been generally found to result in the 5 log pathogen reduction as required by the FDA. An increased UV dose (16.822mJ/cm²), can achieve greater than 5-log reductions of *E. coli,* using a UV-C source was a 8.7 W, 110 V, UV-C germicidal lamp with peak emission at 253.7 nm, having a 505 mm arc length and 15 mm outside diameter (OD) (SBL325, American Ultraviolet Company, Lebanon, IN, USA). The UV lamp was enclosed within a quartz glass sleeve (American Ultraviolet Company, Lebanon, IN, USA) with a 22 mm OD and an air gap of 2.4 mm between UV lamp and sleeve. Perfluoroalkoxy polymer resin (PFA) tubing with 1.6 mm inside diameter (ID) and 3.2 mm OD was selected to wrap around the glass sleeve based on Geveke (2008) that PFA tubing is highly transparent to UV light and has more chemical and heat resistance than polytetrafluoroethylene and fluorinated ethylene propylene.

Another example of a UV reactor suitable for the present invention includes the device shown in European Patent No. EP 1255444, title Sterilization of Liquids using Ultraviolet Light, which describes a device and method for UV sterilization of a turbid liquid.

Optionally, the method further comprises a drying step to form a milk powder according to Step 4 of Figure 5. Conventional drying steps include spray drying, where pasteurized milk is first concentrated in an evaporator to approximately 50% milk solids, then sprayed into a heated chamber where the water almost instantly evaporates. Alternatively, conventional methods for drying milk can be accomplished by drum-drying, where milk is applied as a thin film to the surface of a heated drum, and the dried milk solids removed. However, as will be appreciated, conventional drying steps utilize heat, which has been found by the present inventors to reduce the immune- stimulating activity of milk. Therefore, in the present invention, these high-heat processes have been avoided. Thus, in this embodiment, the drying step is carried out at a temperature of less than about 45 °C, less than about 42 °C, less than about 40 °C, less than about 32 °C, or less than about 0 °C. Methods by which to dry solids at low temperatures are known in the art. These include freeze-drying, low-temperature drying methods. One method includes using a large volume of air compared to the feed of milk, and the drying process is accomplished fairly slowly over an extended period of time. The temperature of the milk during this process may be increased slowly. For example, during this process, the milk may be exposed to a temperature of 42 °C for approximately 2 minutesor less. One example of an appropriate drying process is the one carried out by LiquaDry, Inc. (Hinckley, UT).

Milk contains a number of proteins. Some are known to be immunologically active, although the exact compounds responsible for the full scope of the immunologically active properties of milk remains unknown. Milk proteins include the primary group, the caseins. All other proteins found in milk are called whey proteins. Whey proteins are a mixture of globular proteins, and include all proteins soluble in milk after the pH is dropped to 4.6. The major whey proteins in milk are β-lactoglobulin and a-lactalbumin. Whey proteins denature rapidly at temperatures above 70°C, with thermostability increases from IgG < serum albumin < β-lactoglobulin < α-lactalbumin. The functionality of whey proteins is sensitive to the extent of denaturation.

Caseins have excellent solubility and heat stability above pH 6. The caseins precipitate out of milk at pH 4.6. Casein proteins include a family of related phosphoproteins (α S1, α S2, β , κ) and are a major component of cheese.

Milk proteins according to the invention include, for example, protease inhibitors in the whey portion of the milk (milk serum) proteins such as serpin peptidase inhibitor, clade B (ovalbumin), member 4 (bovine)( SERPINB4); serpin peptidase inhibitor, clade F (alpha-2 antiplasmin, pigment epithelium derived factor), member 2 (bovine) (SERPINF2); serpin peptidase inhibitor, clade D (heparin cofactor), member 1 (bovine), (SERPIND1); serpin peptidase inhibitor, clade B (ovalbumin), member linter-alpha-trypsin inhibitor heavy chain 2 (bovine)( SERPINB1); serpin peptidase inhibitor, clade G (CI inhibitor), member 1 (bovine) (ITIH2), serpin peptidase inhibitor, clade F (alpha-2 antiplasmin, pigment epithelium derived factor), member 1 (bovine)(SERPINFl); inter-alpha-trypsin inhibitor heavy chain 1 (bovine)( ITIH1); serpin peptidase inhibitor, clade A (alpha-1 antiproteinase, antitrypsin), member 3 (bovine)( SERPINA3); serpin peptidase inhibitor, clade C (antithrombin), member 1 (bovine)( SERPINC1); inter-alpha-trypsin inhibitor heavy chain family, member 4 (bovine)(ITIH4); serpin peptidase inhibitor, clade A (alpha-1 antiproteinase, antitrypsin), member 1 (bovine)(SERPINA1); serpin peptidase inhibitor, clade A (bovine) (SERPINA5); SERPINX; cystatin C (bovine)(CST-3); cystatin E/M (bovine)(CST6); this list includes orthologs of the above from another dairy animal.

Milk proteins according to the invention also include complement proteins, which is linked to the protease inhibitor system, as protease inhibitors can influence the activation of the complement system. Complement proteins of interest include CD59 molecule, complement regulatory protein (bovine)( CD59); complement factor D (adipsin)(bovine)(CFD); complement component 6(bovine)(C6); complement component 9(bovine)(C9); complement factor H (bovine)(CFH); complement component 7 (bovine)(C7); complement factor B (bovine) (CFB); complement component 3 (bovine) C3 ; complement component 4 binding protein, alpha (bovine)(C4BPA); CD5 molecule-like (bovine) (CD5L); complement component 4A (bovine)(C4a); complement factor I (bovine) (CFI); this list includes orthologs of the above from another dairy animal.

Milk proteins according to the invention also include other known immune active proteins, such as antibacterial proteins, for example, mucin 15, cell surface associated (bovine) (MUC15); mucin 16, cell surface associated (bovine) (MUC16); cathelicidin 2(bovine)(CATHL2); cathelicidin 4 (bovine)(CATHL4); cathelicidin 1 (bovine)(CATHLl); immunoglobulin J polypeptide, linker protein for immunoglobulin alpha and mu polypeptides (bovine)(IGJ); secreted phosphoprotein 1 (bovine)(SPPl); ribonuclease, RNase A family, 1 (bovine)(RNASEl); butyrophilin, subfamily 1, member A1 (bovine)(BTN1A1); glycosylation-dependent cell adhesion molecule 1 (bovine) (GLYCAM1); clusterin (bovine) (CLU); lactoperoxidase (bovine)(LPO); lactotransferrin (bovine)(LTF); xanthine dehydrogenase (bovine) (XDH); CD14 molecule (bovine) (CD14); this list includes orthologs of the above from another dairy animal.

Other milk proteins according to the present invention include immunoglobulins, such as polymeric immunoglobulin receptor (bovine) (PIGR); joining chain of multimeric IgA and IgM (bovine) (IGJ); Ig kappa chain (bovine)(IGK); immunoglobulin lambda locus (IGL); immunoglobulin M (bovine) (IGM); this list includes orthologs of the above from another dairy animal.

The nonfat milk fraction together with the composition comprising MFGM components, is then subjected to UV-C treatment to accomplish pasteurization as described elsewhere herein. In one embodiment, the nonfat milk fraction is treated after collection and before addition of a composition comprising MFGM components. The composition comprising MFGM components, or source of MFGM components such as whey or buttermilk, may be UV-C treated prior to additional processing, as UV-C treatment efficacy is enhanced when the liquid has a low viscosity and is more transparent.

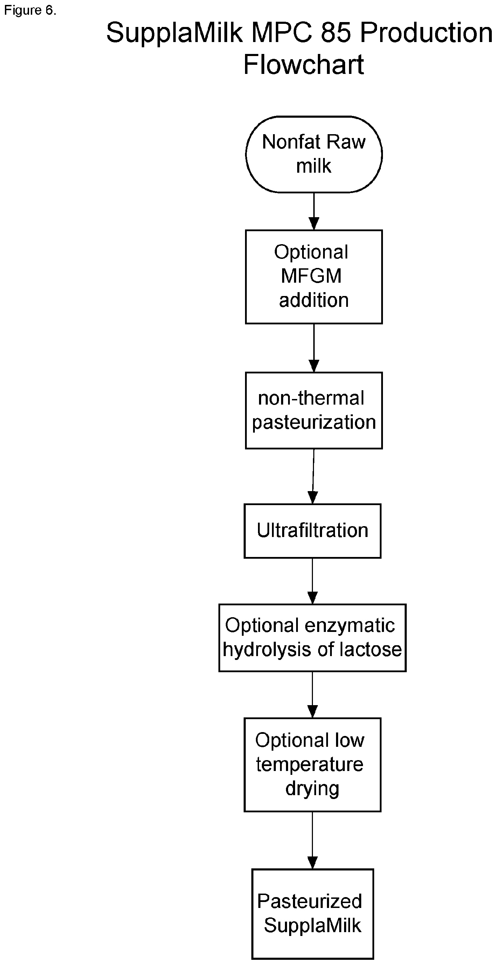

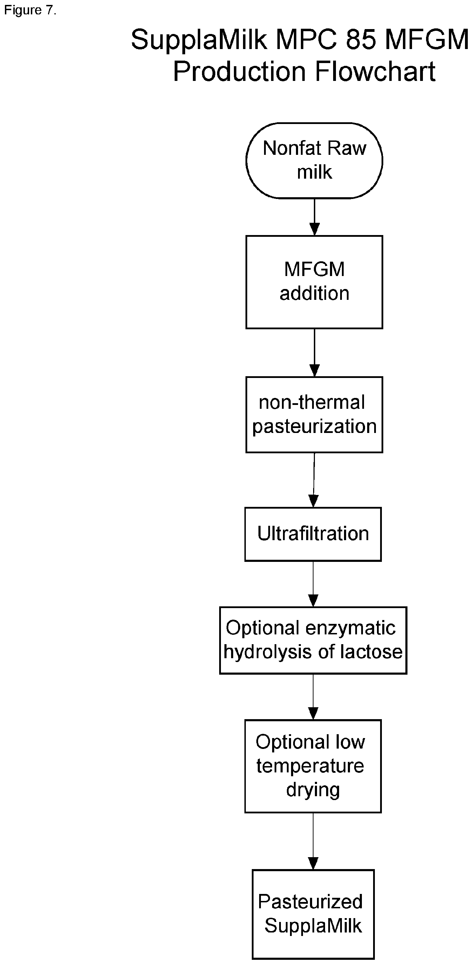

Figure 6 shows a flow chart of an embodiment of the present invention for the production of MPC 85 milk or other concentrated milk product of the present invention (also called SUPPLAMILK in the flowcharts). Milk protein concentrates are concentrated preparations of milk proteins that contain milk proteins in the same or a similar ratio to each other as milk or nonfat milk. The processes to prepare milk protein concentrates allow the proteins to stay in their native state and largely un-denatured. Milk protein concentrates may be prepared by filtration processes, such as microfiltration, ultrafiltration and diafiltration. In one embodiment, both ultrafiltration and diafiltration are used. These processes use membrane separation technology to remove the majority of lactose and soluble minerals while retaining the milk protein. MPCs are manufactured ranging in protein content from 42 to 90 percent. Common MPC products are MPC 42, MPC 70, MPC 80, MPC 85 and MPI (which typically contains 90 percent or more protein by weight). MPC is typically made from skim milk, resulting in fat levels of less than 3 percent. As a rule of thumb, as the protein content of MPCs increases, the lactose levels decrease. For example, NFDM contains about 34 to 36 percent protein and 52 percent lactose, while MPC 42 contains 42 percent protein and 46 percent lactose, and MPC 80 contains 80 percent protein and 5 to 6 percent lactose, respectively. MPCs with even higher protein content also are available. They are generally known as MPIs with a minimum of 90 percent protein (generally in the range of 90 to 91 percent protein by weight) and micellar casein concentrates with 93 to 94 percent protein. Therefore, in Figure 6, the flowchart shows one embodiment of the production of a milk prepared by the processes of the present invention. Raw milk is used to prepare a nonfat raw milk as discussed elsewhere herein, and a composition comprising MFGM as described hereinis added. A non-thermal pasteurization step may be performed, followed by the filtration step, e.g., ultrafiltration, to produce the MPC of the desired concentration and lactose level. Optionally, remaining lactose may be removed via enzymatic hydrolysis, as described elsewhere herein, followed by optional low-temperature drying as described herein to produce the milk product of the present invention. Figure 7 shows an embodiment of the processes of the present invention incorporating a composition comprising MFGM to make an MPC 85 preparation.

The suitability of batches of milk prepared by the processes of the invention may be determined by measurement of the level of and/or activity of one or more of the proteins identified hereinabove, compared to raw milk. Optionally, the milk which has been treated according to the methods of the instant invention to result in an immune-active milk composition has at least 100%, at least 99%, at least 98%, at least 97%, at least 96%, at least 95%, at least 94%, at least 93%, at least 92%, at least 91%, at least 90%, at least 89%, at least 88%, at least 87%, at least 86%, at least 85%, at least 84%, at least 83%, at least 82%, at least 81%, at least 80%, at least 79%, at least 78%, at least 77%, at least 76%, at least 75%, at least 74%, at least 73%, at least 72%, at least 71%, at least 70%, at least 69%, at least 68%, at least 67%, at least 66%,at least 65%, at least 64%, at least 63%, at least 62%, at least 61%,at least 60%, at least 59%, at least 58%, at least 57%, at least 56%,at least 55%, at least 54%, at least 53%, at least 52%, at least 51%,at least 50%, at least 49%, at least 48%, at least 47%, at least 46%, at least 45%, at least 40%, at least 39%, at least 38%, at least 37%, at least 36%, at least 35%, at least 34%, at least 33%, at least 32%, at least 31%, at least 30%, at least 29%, at least 28%, at least 27%, at least 26%, at least 25%, or at least 20% of the amount of, and/or activity of, a milk protein of the invention, as compared to raw milk, either as a standardized raw milk or to a specific sample of raw milk. In one embodiment, the immune-active milk composition has at least 90% of the level of, or activity of, at least one protein described herein.

Resultant immune active milk compositions may be stored prior to consumer use by methods known in the art, i.e., methods known for storage of liquid milk or powdered milk. In one embodiment, the liquid milk and powdered milk formulations are stored under refrigeration.

The present invention also includes an immune active milk composition of the present invention according to Figures 5, 6, and 7 (called SUPPLAMILK in the figure). This composition, in one embodiment, comprises a UV-C treated nonfat milk. In another embodiment, the immune active milk composition of the present invention includes a UV-C treated nonfat milk and also a composition comprising MFGM components, pasteurized either by UV-C pasteurization or by other non- thermal pasteurization methods, in either liquid or dried form. The compositions of the present invention may also be milk protein concentrates, as discussed herein. The dried composition, in one embodiment, is dried using low-temperature drying methods.

Immune-active milk compositions of the present invention may be used to treat childhood asthma. Dosage would be similar to normal intake of milk at each age, e.g., for children, about 200-300 mL per day is a normal intake of milk, which is equivalent to about 24 to 35 g of the powdered milk composition of the invention, for example. Suitable dosages include between about 0.1 g and about 1000 g, about 1 g to about 500 g, from about 5 g to about 100 g, from about 10 g to about 80 g, from about 20 g to about 60 g, or about 24 g to about 35 g; or at least about 2 g, at least about 2 g, at least about 6 g, at least about 8 g, at least about 10 g, at least about 12 g, at least about 14 g, at least about 16 g, at least about 18 g, at least about 20 g, at least about 22 g, at least about 24 g, at least about 26 g, at least about 28 g, at least about 30 g, at least about 32 g, at least about 34 g, at least about 36 g, at least about 40 g or higher amounts. Suitable individuals for treatment using the immune-active milk compositions are children, such as those under age 1, those under age 2, or older children, as well as adults of any age.

Administration can be on an as-needed or as-desired basis, for example, once- monthly, once-weekly, daily, or more than once daily. Similarly, administration can be every other day, week, or month, every third day, week, or month, every fourth day, week, or month, and the like. Administration can be multiple times per day. When utilized as a supplement to ordinary dietetic requirements, the composition may be administered directly to the individual or otherwise contacted with or admixed with daily feed or food. When utilized as a daily feed or food, administration will be well known to those of ordinary skill.

Administration can also be carried out on a regular basis, for example, as part of a treatment regimen in the individual. A treatment regimen may comprise causing the regular ingestion by the individual of an inventive composition in an amount effective to enhance immune function or reduce dysfunctional immune function in the individual. Regular ingestion can be once a day, or two, three, four, or more times per day, on a daily or weekly basis. Similarly, regular administration can be every other day or week, every third day or week, every fourth day or week, every fifth day or week, or every sixth day or week, and in such a regimen, administration can be multiple times per day. The goal of regular administration is to provide the individual with optimal dose of an inventive composition, as exemplified herein.

The compositions provided herein are, in one embodiment, intended for "long term" consumption, sometimes referred to herein as for 'extended' periods. "Long term" administration as used herein generally refers to periods in excess of one month. Periods of longer than two, three, or four months comprise one embodiment of the instant invention. Also included are embodiments comprising more extended periods that include longer than 5, 6, 7, 8, 9, or 10 months. Periods in excess of 11 months or 1 year are also included. Longer terms use extending over 1, 2, 3 or more years are also contemplated herein. In some individuals, it is envisioned that the individual would continue consuming the compositions for the remainder of the individual's life on a regular basis. "Regular basis" as used herein refers to at least weekly, dosing with or consumption of the compositions. More frequent dosing or consumption, such as twice or thrice weekly are included. Also included are regimens that comprise at least once daily consumption. The skilled artisan will appreciate that the reduction of or prevention of a dysfunctional immune response may be a valuable measure of dosing frequency. Any frequency, regardless of whether expressly exemplified herein, that allows maintenance of a functional immune response within acceptable ranges can be considered useful herein.

As used herein, the term "oral administration" or "orally administering" means that the mammal ingests, or a caretaker is directed to feed, or does feed, the individual one or more of the compositions described herein. The immune-active milk compositions of the present invention may be used as a food product, which will include foods and nutrients intended to supply necessary dietary requirements of a human being as well as other human dietary supplements. In a one embodiment, the food products formulated for human consumption are complete and nutritionally balanced, while in others they are intended as nutritional supplements to be used in connection with a well-balanced or formulated diet.

In another embodiment, the composition is a food supplement, such as drinking water, beverage, liquid concentrate, gel, yogurt, powder, granule, paste, suspension, chew, morsel, treat, snack, pellet, pill, capsule, tablet, or any other delivery form including milk, powdered milk, or incorporated into any foods containing dairy as known in the art. The nutritional supplements can be specially formulated for consumption by a particular species or even an individual mammal, such as companion mammal, or a human. In one embodiment, the nutritional supplement can comprise a relatively concentrated dose of the immune-active milk composition such that the supplement can be administered to the individual in small amounts, or can be diluted before administration to an individual. In some embodiments, the nutritional supplement or other MCT-containing composition may require admixing with water or the like prior to administration to the individual, for example to adjust the dose, to make it more palatable, or to allow for more frequent administration in smaller doses.

In some embodiments, the individual is specifically a human. Other individuals include mammals such as companion animals, such as a dog or cat.

While various aspects and features of certain embodiments have been summarized above, the following detailed description illustrates a few embodiments in further detail to enable one of skill in the art to practice such embodiments. The described examples are provided for illustrative purposes and are not intended to limit the scope of the invention.

### EXAMPLES

The following examples are provided for illustrative purposes only and are not intended to limit the scope of the invention.

### Example 1 ― Not part of the claimed invention

### UV-C treatment of raw milk

Milk was UV-C pasteurized at 1000 J/liter. Samples of raw milk and two separate UV-C samples were compared to standard pasteurized whole milk and pasteurized and spray dried whole milk powder.

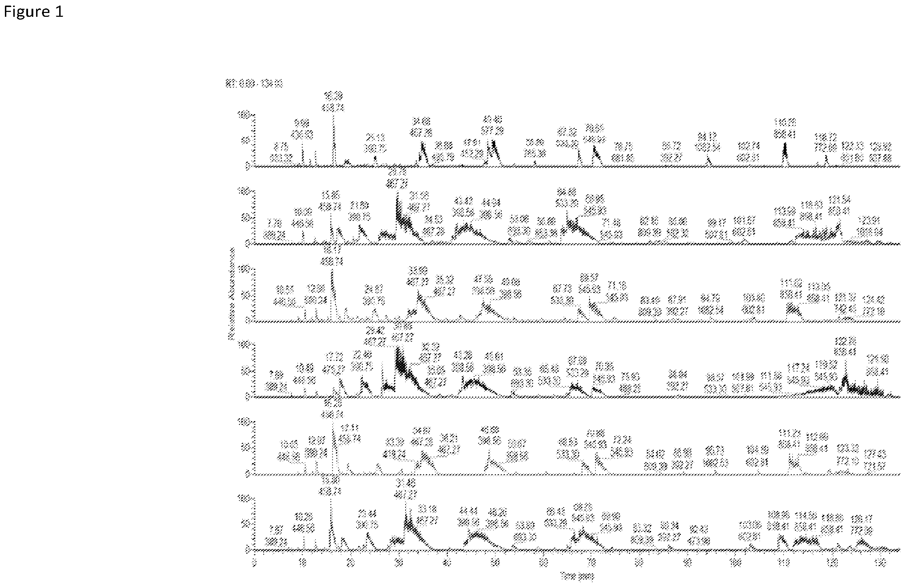

LC/MS chromatography. Figure 1 shows LC/MS TIC chromatograms of the raw samples (first and second chromatograms) and 1000 J samples (third to sixth chromatograms.) Using an overall statistical analysis of protein change using Perseus software (available from MaxQuant) which is useful to analyze expression proteomics, posttranslational modifications, interaction proteomics, machine learning for biomarker discovery, time series analysis and cross- omics data analysis. No proteins were statistically significantly different between the raw v. 1000 J samples. Following this test, the samples grouped based on gene ontology (biological function) and plotted the difference between the two samples. Ultracentrifugation at pH 4.6, to make milk serum was performed on the raw milk and the 1000 J treated milk, to remove denatured proteins.

The supernatant was prepared using filter-aided sample preparation (FASP) was employed, resulting in two samples obtained from the raw milk and 4 samples from the 1000 J treated milk. Supernatants were prepared in the presence of high concentrations of detergent. Disulfide bridges were reduced with dithiothreitol (DTT). Detergent micelles and protein detergent complexes were dissociated in the presence of 8 M urea. The detergent, DTT and other low-molecular-weight components were removed by utrafiltration (Microcon units) facilitated by centrifugation. Thiols were carboxyamidomethylated with iodoacetamide (IAA) and excess reagent was removed by ultrafiltration. In repeated washes with 8 M urea any remaining detergent was depleted from the proteins. The protein suspension was digested with endoproteinase, and the resulting peptides were collected as a filtrate. High-molecular-weight molecules including the endopro tease are retained on the filter. When nuclei or total cell ly sates are processed in the units, DNA is retained on the filter.

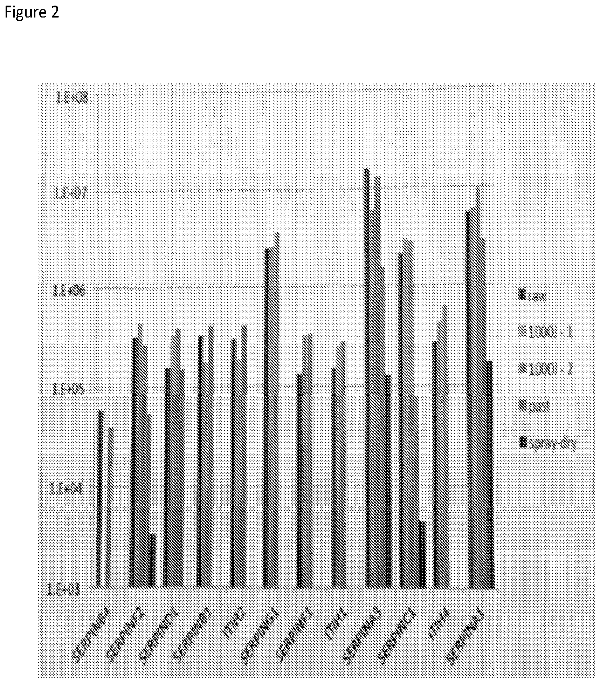

Protease inhibitors have been linked to allergy and asthma. It was found that protease inhibitors, a heat-sensitive group of milk proteins, significant degradation is seen for the pasteurized and spray-dried samples. It was found that proteins (shown by their gene code) were treated, all the data points showed identical intensities for the samples, but significant degradation was observed in the pasteurized and spray dried samples. While spray-dried samples and pasteurized samples show decreases, none of the proteins tested showed a clear decreasing trend after the UV-C treatment. See Figure 2.

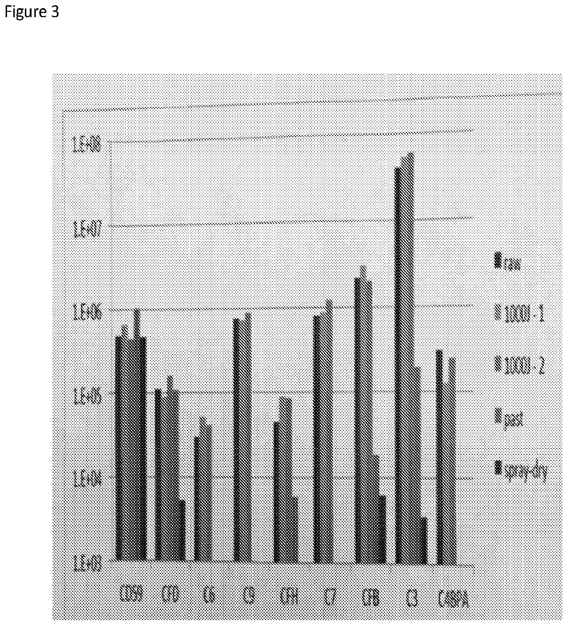

Complement proteins. Complement system is an innate immune system. C3 is one of the central components of this system, and also is the most abundant protein of this system in milk. The complement system is linked to the protease inhibitor system, as protease inhibitors can influence activation of the complement system. Significantly lower levels of complement proteins are shown in the pasteurized and spray-dried samples. While spray-dried samples and pasteurized samples show decreases, none of the proteins tested showed a clear decreasing trend after the UV-C treatment. See Figure 3.

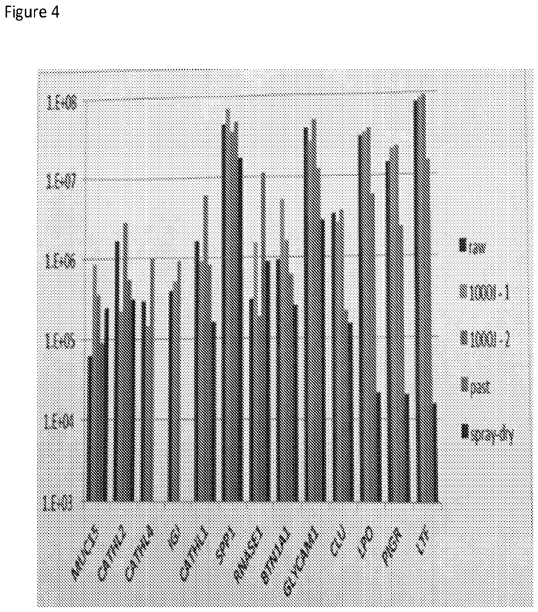

Other immune-active proteins were also tested. See Figure 4. SPP1 (osteopontin) and LTF (lactoferrin) are the most abundant immunologically- active proteins in milk. Lactoferrin is especially known for its temperature sensitivity, as it easily denatures even under low-pasteurization conditions. While spray-dried samples and pasteurized samples show decreases, none of the proteins tested showed a clear decreasing trend after the UV-C treatment.

The conclusions drawn from this study show that overall protein, and specific known immune-active proteins, are not decreased after 1000 J UV-C.

### Example 2

Preparation of UV-C milk product. Raw cow's milk was obtained, and separated into a nonfat milk fraction and a fat fraction through centrifugation according to standard methods. The resultant nonfat milk had between 0.1% and 0.3% fat. The nonfat milk fraction was enzymatically treated to hydrolyze lactose using LACTOZYM PURE (Novozym, Denmark), according to manufacturer instructions.

A composition comprising milk fat globule membrane components (MFGM) obtained from raw buttermilk by methods known in the art and combined with the raw nonfat milk. The MFGM supplemented nonfat milk was pasteurized with a SurePure SP40 turbulent flow UV-C pasteurizing system (obtainable from SurePure, AG., Capetown, South Africa; see U.S. 6,916,45); the milk is processed in this system two times to ensure achievement of 2000 J/1.

The solution comprising the UV-C pasteurized nonfat milk was dried into a powder at low temperatures (below 45°C) or through freeze-drying. Procedures used exposed the milk to a maximum temperature of 42°C for less than 2 minutes. Alternatively, an MPC 85 was prepared. The final fat concentration of the resultant product is approximately 1-1.5%.

A milk prepared according to the present invention, according to the process of Figure 6, without the optional MFGM addition and without the optional enzymatic hydrolysis of lactose, and with the optional low-temperature drying, has the following characteristics:

| **Property** | **Specification** | **Test Method** |
|---|---|---|
| Color | Off white | Visual |
| Moisture | <5.0% | AOAC 930.15 |
| Fat | <1.5% | |
| Protein | >85% | |
| Lactose | Not detected | |
| Total sugars | <3.8% | |

| **Property** | **Specification** | **Test Method** |
|---|---|---|
| 5PC | <10,000 CFU/g | USP<2021> |
| Coliform | <2,500 MPN/g | AOAC 966,24 |
| E. Coli | <3 MPN/g | AOAC 988,19 |
| Staphylococcus | Absent / 10 g | USP <2022> |
| Salmonella | Absent / 25 g | USP <2022> |
| Yeast | <5,000 CFU/g | USP <2021> |
| Mold | <5,000 CFU/g | USP <2021> |
| Listeria | ≤1 cell / 25 g | FDA BAM |

### Example 3

A composition prepared according to the methods of Example 2 is obtained and analyzed by the methods of Example 1. The identified proteins were compared between the composition of the invention (called SUPPLAMILK NF) and nonfat milk powder, obtained by conventional methods. As can be seen, the amount of the measured proteins remaining (by MS) is significantly higher for all proteins other than SPP1.

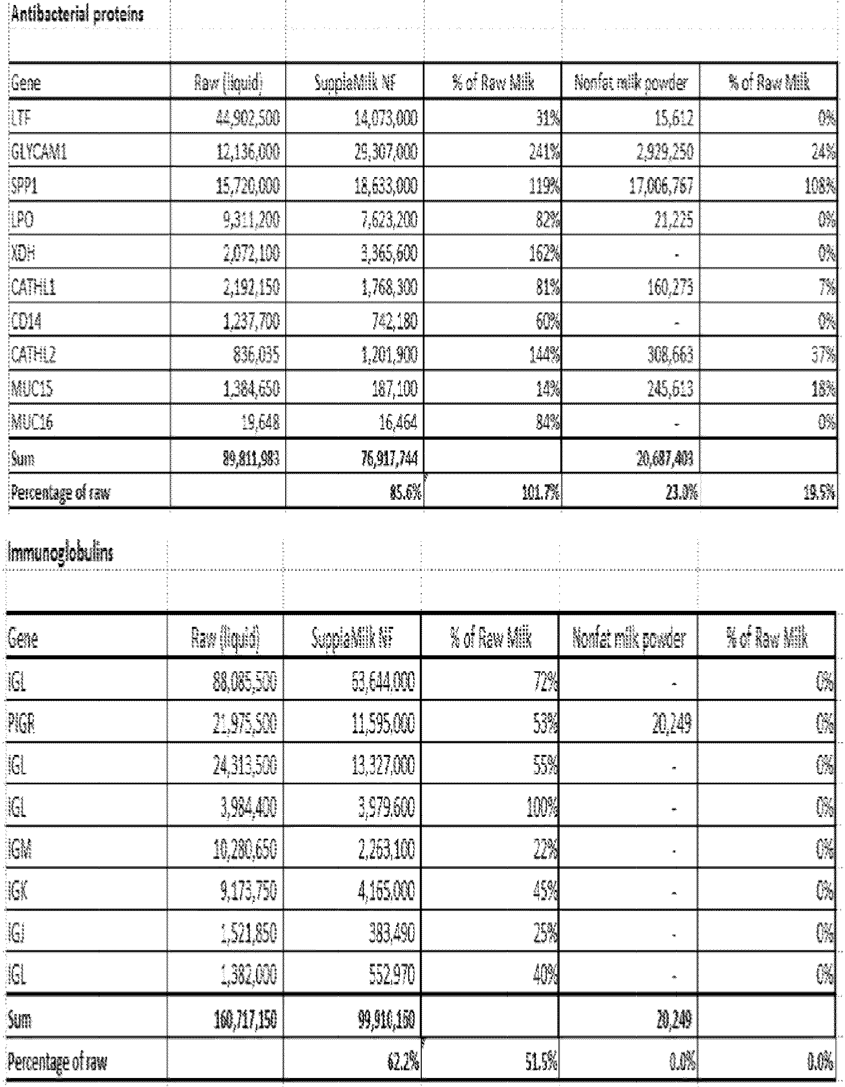

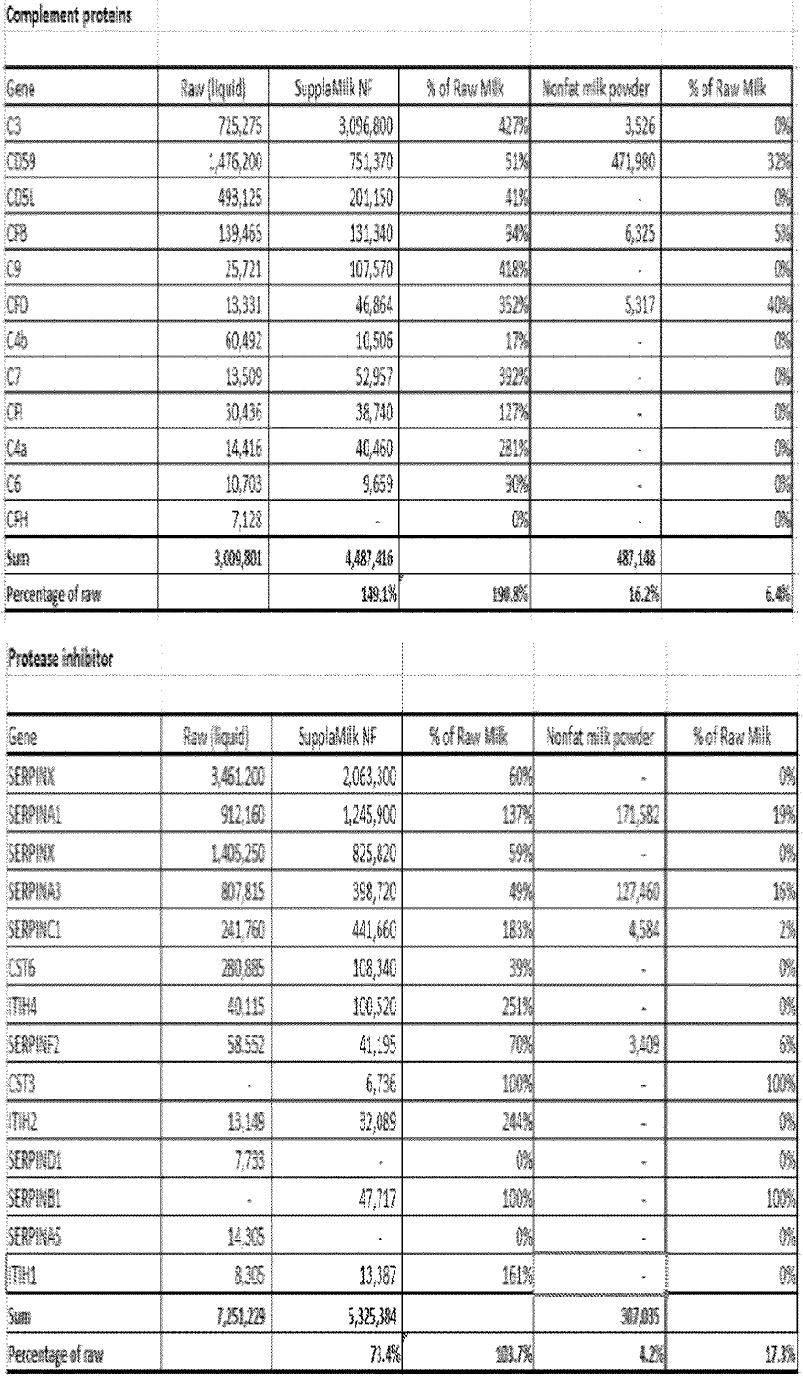

Many clinical trials have proven raw cow's milk consumption sufficiently activates human immune systems to significantly reduce allergy and respiratory infection development in children. Additional studies have shown milk protein consumption enhances elderly vaccine response. Milk products prepared in accordance with the present invention retain most of the immune active proteins found in raw milk, while standard nonfat milk powder does not. Many researchers believe these immune active proteins are responsible for raw milk's ability to stimulate immune responses. The below table shows a summary for each protein class. Overall, the milk product of the present invention contains a much greater percentage of each protein, compared with raw milk, than nonfat milk protein prepared by conventional methods.

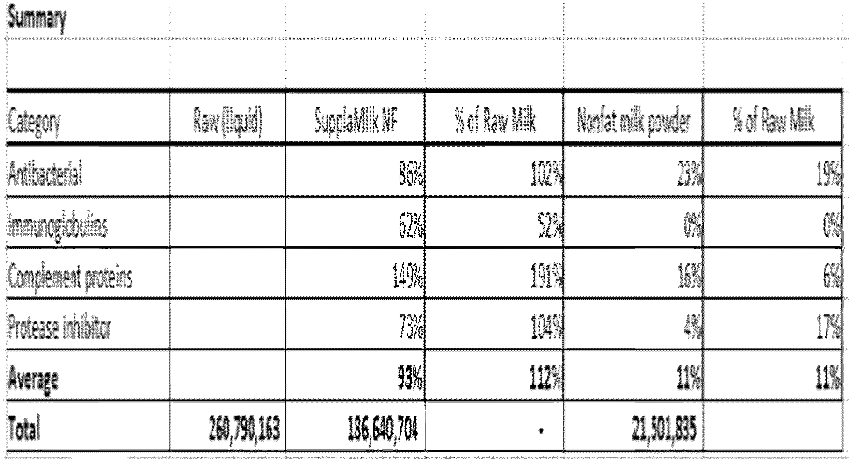

### Example 4

MARINA - large weaned infant study comparing intervention with SupplaMilk to pasteurized, homogenized and spray dried whole milk powder for respiratory infection, allergies and asthma. The immune-active milk composition (Intervention) to be tested is prepared by the following steps: USD A Grade A whole milk, non-homogenized, but continuously stirred during storage; pasteurized using turbulent flow ultraviolet light in the "C" bandwidth (200-280 nm) with 1,100 J of exposure per liter of milk; pathogen testing of the UV-C pasteurized milk and dried into a powder containing less than 5% moisture using a low temperature drying system with maximum temperature exposure of 41°C (106°F). This is compared to (comparator) USD A Grade A milk from same batch as Intervention, ultra-high temperature pasteurized, homogenized and spray dried to less than 5% moisture using conventional high temperature spray drying techniques. The immune active milk composition is found to found to enhance infant immunity compared to milk not treated by methods of the invention.

## Claims

1. A method to produce an immune-active milk product comprising:
a. obtaining a nonfat milk fraction from raw (unpasteurized) milk;
b. obtaining a composition comprising milk fat globule membrane components (MFGM) from buttermilk;
c. combining the composition comprising MFGM components with the nonfat milk fraction; and
d. pasteurizing the product of step c) by application of UV-C light to prepare the immune-active milk product.

2. The method of any of claim 1, further comprising concentrating the product following the UV-C pasteurization step, preferably wherein the concentration step is accomplished by ultrafiltration and diafiltration to create a milk protein concentrate as the immune-active milk product.

3. The method of claim 2, wherein the concentration step results in a protein concentration of 85% by weight.

4. The method of any of claims 1-3, further comprising: treating the nonfat milk fraction to hydrolyze the lactose.

5. The method of claim 1, further comprising drying the immune-active milk product to a powder, preferably wherein the drying step is a freeze-drying step, preferably wherein the drying step occurs at below 45 °C.

6. The method of claim 5 wherein the powdered immune-active milk product is dryblended with a dried composition comprising MFGM components.

7. The method of any preceding claim, wherein the UV-C light is applied at between 1000 and 2000 J/liter.

8. The method of any preceding claim, wherein the raw milk composition is obtained from dairy cows.

9. An immune-active milk product produced by the method of any preceding claim.

10. The immune-active milk product obtained by the process of any preceding claim for use in the treatment of childhood asthma.

## Patentansprüche

1. Verfahren zur Herstellung eines immunaktiven Milchprodukts, das Folgendes umfasst:
a. Gewinnen einer fettfreien Milchfraktion aus (nicht pasteurisierter) Rohmilch;
b. Gewinnen einer Zusammensetzung, die Milchfettkügelchenmembran-Komponenten (MFGM) aus Buttermilch umfasst;
c. Vereinigen der Zusammensetzung, die MFGM-Komponenten umfasst, mit der fettfreien Milchfraktion; und
d. Pasteurisieren des Produkts von Schritt c) durch Anwendung von UV-C-Licht, um das immunaktive Milchprodukt herzustellen.

2. Verfahren nach Anspruch 1, das weiter das Konzentrieren des Produkts nach dem UV-C-Pasteurisierungsschritt umfasst, bevorzugt wobei der Konzentrationsschritt durch Ultrafiltration und Diafiltration erreicht wird, um ein Milchproteinkonzentrat als das immunaktive Milchprodukt zu erzeugen.

3. Verfahren nach Anspruch 2, wobei der Konzentrationsschritt zu einer Proteinkonzentration von 85 Gewichts-% führt.

4. Verfahren nach einem der Ansprüche 1-3, das weiter Folgendes umfasst:
Behandeln der fettfreien Milchfraktion, um die Laktose zu hydrolysieren.

5. Verfahren nach Anspruch 1, das weiter das Trocknen des immunaktiven Milchprodukts zu einem Pulver umfasst, bevorzugt wobei der Trocknungsschritt ein Gefriertrocknungsschritt ist, bevorzugt wobei der Trocknungsschritt unterhalb von 45 °C erfolgt.

6. Verfahren nach Anspruch 5, wobei das pulverisierte immunaktive Milchprodukt mit einer getrockneten Zusammensetzung, die MFGM-Komponenten umfasst, trocken gemischt wird.

7. Verfahren nach einem vorstehenden Anspruch, wobei das UV-C-Licht bei zwischen 1000 und 2000 J/Liter angewendet wird.

8. Verfahren nach einem vorstehenden Anspruch, wobei die Rohmilchzusammensetzung von Milchkühen gewonnen wird.

9. Immunaktives Milchprodukt, das durch das Verfahren nach einem vorstehenden Anspruch hergestellt wird.

10. Immunaktives Milchprodukt, das durch das Verfahren nach einem vorstehenden Anspruch gewonnen wird, für die Verwendung bei der Behandlung von Kindheitsasthma.

## Revendications

1. Méthode de production d'un produit de lait immuno-actif, comprenant :
a. l'obtention d'une fraction de lait dégraissée à partir de lait cru (non pasteurisé) ;
b. l'obtention d'une composition comprenant des composants de membrane de globule gras du lait (MFGM) à partir de babeurre ;
c. la combinaison de la composition comprenant les composants de MFGM avec la fraction de lait dégraissée ; et
d. la pasteurisation du produit de l'étape c) par l'application de lumière UV-C afin de préparer le produit de lait immuno-actif.

2. Méthode selon la revendication 1, comprenant en outre la concentration du produit suite à l'étape de pasteurisation par UV-C, préférablement où l'étape de concentration est mise en œuvre par ultrafiltration et diafiltration afin de créer un concentré de protéines de lait en tant que produit de lait immuno-actif.

3. Méthode selon la revendication 2, dans laquelle l'étape de concentration conduit à une concentration en protéines de 85% en poids.

4. Méthode selon l'une quelconque des revendications 1-3, comprenant en outre le traitement de la fraction de lait dégraissée afin d'hydrolyser le lactose.

5. Méthode selon la revendication 1, comprenant en outre le séchage du produit de lait immuno-actif en une poudre, préférablement où l'étape de séchage est une étape de lyophilisation, préférablement où l'étape de séchage a lieu à moins de 45°C.

6. Méthode selon la revendication 5, dans laquelle le produit de lait immuno-actif en poudre est mélangé à sec avec une composition séchée comprenant des composants de MFGM.

7. Méthode selon l'une quelconque des revendications précédentes, dans laquelle la lumière UV-C est appliquée à entre 1000 et 2000 J/litre.

8. Méthode selon l'une quelconque des revendications précédentes, dans laquelle la composition de lait cru est obtenue à partir de vaches laitières.

9. Produit de lait immuno-actif préparé par la méthode selon l'une quelconque des revendications précédentes.

10. Produit de lait immuno-actif obtenu par le procédé selon l'une quelconque des revendications précédentes, pour une utilisation destinée au traitement de l'asthme chez l'enfant.
